# EUROPÄISCHE PATENTSCHRIFT

(11) **EP 4 154 846 B1**
(45) Veröffentlichungstag und Bekanntmachung des Hinweises auf die Patenterteilung: **29.10.2025**
(21) Anmeldenummer: 21198634.4
(22) Anmeldetag: 23.09.2021
(51) Int. Cl.: A61C 17/10, A61C 17/06

(54) **ZAHNÄRZTLICHE SAUGVORRICHTUNG**
DENTAL SUCTION DEVICE
DISPOSITIF D'ASPIRATION DENTAIRE

(43) Veröffentlichungstag der Anmeldung: 29.03.2023
(73) Patentinhaber: Ivoclar Vivadent AG, 9494 Schaan (LI)
(72) Erfinder: LICHTENSTEIGER, Markus, 9462 Montlingen (CH); BÜCHEL, Adrian, 9498 Planken (LI)
(74) Vertreter: Baldus, Oliver

(56) Entgegenhaltungen:
- US-A1- 2016 038 348
- US-A1- 2016 345 815
- US-A1- 2021 038 354
- US-B2- 7 335 023
- US-B2- 8 231 384

## Beschreibung

Die Erfindung betrifft einen Speichelsauger.

Derartige Speichelsauger sind seit langem bekannt und dienen dazu, Speichel und gegebenenfalls sonstige Flüssigkeiten aus dem Mund eines Patienten abzusaugen. Typischerweise weist ein derartiger Speichelsauger einen Saugschlauch und ein Saugelement auf. Der Saugschlauch ist bevorzugt gebogen und wird mit seinem gebogenen Bereich in den Mund des Patienten eingehängt.

Wenn im oralen Bereich des Patienten abgesaugt werden soll, erstreckt sich der Saugschlauch über die Unterkiefer-Zahnreihe und das Saugelement soll dann an der tiefsten Stelle im Mund des Patienten die erwünschte Saugwirkung entfalten. Das Saugelement ist typischerweise als Schwamm ausgebildet und umgibt als solcher den Saugschlauch an seinem oralen Ende. Gegebenenfalls ist der Saugschlauch dort mit kleinen Löchern versehen, weist jedoch jedenfalls ein offenes Ende auf.

Der Schwamm muss typischerweise separat hergestellt werden. Er wird auf das Ende des Saugschlauch aufgesteckt. Er ist so bemessen, dass er dort einen festen Sitz hat. Damit soll verhindert werden, dass er im Mund des Patienten versehentlich vom Ende des Saugschlauches rutscht und dann als Fremdkörper im Mund des Patienten verbleibt.

Typischerweise haben schwammartige Körper aus Kunststoff keine lange Lebensdauer und verlieren im Laufe der Zeit auch ihre Elastizität. Daher kann es passieren, dass ein derartiger Schwamm sich doch vom Saugschlauch löst, wenn er die empfohlene Lebensdauer überschritten hat.

Um dies zu verhindern, ist es vorgeschlagen worden, kleine Widerhaken auf dem Saugschlauch anzubringen, die ein Abrutschen verhindern sollen.

Ferner ist es vorgeschlagen worden, den Schwamm auf den Saugschlauch aufzukleben. Dann lässt er sich jedoch nicht mehr von dem Saugschlauch lösen, und der Saugschlauch muss häufig ausgetauscht werden, was unwirtschaftlich ist.

Es ist auch bekannt geworden, kurzerhand auf den Schwamm zu verzichten. Damit erübrigen sich die mit dem Schwamm verbundenen Probleme, aber der vergleichsweise harte Saugschlauch wird gerade unter der Zunge als unangenehm empfunden.

Man hat daher versucht, den Saugschlauch mit einem Zungenhalter zu kombinieren, der die Zunge sowohl von den Zähnen als auch von dem
Saugschlauch selbst fernhält. Hierdurch wird allerdings der Druck auf das orale Weichgewebe durch das gegenüberliegende, untere Ende des Saugschlauch noch vergrößert, was ebenfalls als unangenehm empfunden wird.

Derartige Lösungen sind seit langem bekannt, beispielsweise aus der DE 1 063 329 B1.

Heutzutage sind derartige Speichelsauger ein Massenartikel und werden in großem Umfang hergestellt und verkauft. Daher spielen auch die Kosten eine Rolle.

Die Druckschrift US 2016/038348 A1 betrifft einen oralen Hygienetupfer für einen Patienten.

Die Druckschrift US 2016/345815 A1 betrifft einen Zahnspiegel, der zur Verwendung bei zahnärztlichen und chirurgischen Eingriffen vorgesehen ist, damit ein Zahnarzt den Zustand der Mundhöhle oder der Zähne des Patienten beobachten kann.

Die Druckschrift US 8,231,384 B2 betrifft Dentalwerkzeuge, die mit einem Saugsystem, gekoppelt werden können, um während eines zahnärztlichen Eingriffs Materie aus dem Mund eines Patienten zu evakuieren.

Die Druckschrift US 2021/038354 A1 betrifft eine Saugvorrichtung für die intraorale Anwendung mit einem Saugrohr und einem Zungenschutz.

Die Druckschrift US 7,335,023 B2 betrifft eine Aspiratoranordnung zur Verwendung bei dentalen oder medizinischen Verfahren zur Entfernung von Körperflüssigkeit und Rückständen vom Ort einer Behandlung.

Daher liegt der Erfindung die Aufgabe zugrunde, einen Speichelsauger zu schaffen, der kostengünstig herzustellen ist, sicher in der Anwendung ist und vom Patienten als angenehm empfunden wird. Diese Aufgabe wird erfindungsgemäß durch Anspruch 1 gelöst. Vorteilhafte Weiterbildungen ergeben sich aus den Unteransprüchen.

Ein erfindungsgemäße Speichelsauger zeichnet sich dadurch aus und, dass er ein besonderes Kunststoff-Formteil aufweist.

Dieses wird bevorzugt auf das Ende eines Saugschlauchs aufgesteckt.

Ein Kunststoff-Formteil ist für sich genommen typischerweise formstabil und daher eher hart. Ein solches Formteil würde vom Patienten als unangenehm empfunden.

Erfindungsgemäß ist das Formteil als Mehrkomponenten-Spritzgussteil hergestellt. Diesel Maßnahme hat den überraschenden Vorteil, dass eine Hartkomponente und eine Weichkomponente in einem hergestellt werden können, ohne dass zusätzliche Kosten entstehen. Die Hartkomponente kann für die Kupplung an den Saugschlauch verwendet werden. Die Weichkomponente weist mindestens eine Saugöffnung oder Saugpore auf. Sie kann an dem Weichgewebe des Patienten anliegen, oral, aber auch vestibulär, ohne dass dies vom Patienten als unangenehm empfunden wird.

Durch die Weichkomponente hindurch lässt sich der Speichel des Patienten sehr effizient absaugen.

Die Hartkomponente eignet sich hingegen besonders gut für den Anschluss an den Saugschlauch. Durch den festen Sitz ist sichergestellt, dass das Saugelement nicht verlorengehen kann. Die Hartkomponente bildet eine Kupplung aus, mit welcher Sie zum Schlauch hin gekoppelt werden kann. Die Kupplung ist langlebig, zumindest im Vergleich mit einem Schwamm.

Über die Kupplung lässt sich das Kunststoff-Formteil aber auch lösen, wenn es gezielt vom Schlauch getrennt werden soll. Dies kann beispielsweise dann der Fall sein, wenn das Formteil desinfiziert werden soll oder wenn es ausgetauscht werden soll.

In vorteilhafter Ausgestaltung ist ein Adapter vorgesehen, der unlösbar mit dem Saugschlauch verbunden ist. Bei dieser Ausgestaltung kuppelt die Kupplung dann über lösbare Rastvorsprünge an den Adapter an. Diese Ausgestaltungen bietet eine noch größere Sicherheit gegen einen Verlust des Formteils.

Erfindungsgemäß ist es jedenfalls vorgesehen, dass die Weichkomponente einen Saugbereich ausbildet. Der Saugbereich hat bevorzugt eine Vielzahl von Saugporen. Bevorzugt weist die Hartkomponente eine Form auf, die sich dreidimensional im Raum erstreckt und einen Bereich aufspannt, der von der Weichkomponente, insbesondere deren Saugbereich, abgedeckt ist. Die Weichkomponente ist dementsprechend nicht formstabil, wird jedoch vorteilhafterweise von der Hartkomponente in Form gehalten.

Durch das Mehrkomponenten-Spritzgießen sind die beiden Komponenten sehr fest und gleichsam einstückig miteinander verbunden.

Die Herstellung erfolgt in an sich bekannter Weise so, dass eine Komponente, beispielsweise die härtere, mit einer Spritzdüse und die andere Komponente, beispielsweise die weichere, mit einer anderen Spritzdüse eingespritzt wird. Es werden insofern 2 Spritzdüsen oder Spritzeinheiten verwendet. Es ist jedoch lediglich eine Schließeinheit erforderlich.

Die beiden Komponenten können damit kostengünstig mit nur einem Werkzeug und in einem Arbeitsgang hergestellt werden.

Bevorzugt ist durch die Hartkomponente eine Hohlkammer vorgegeben. Die Hohlkammer ist länglich und weist an einem Ende einer Auslassöffnung auf und ist am anderen Ende geschlossen. Ein großer Teil der Hohlkammer wird von der Weichkomponente eingenommen, bevorzugt jedoch etwas weniger als die Hälfte. Die Weichkomponente erstreckt sich entlang einer Längsseite der Hohlkammer.

Die Auslassöffnung ist an den Saugschlauch angeschlossen. Durch ihn wird die Hohlkammer unter Unterdruck gesetzt. Durch den Unterdruck wird durch die Saugporen Speichel angesaugt.

Bevorzugt ist die Weichkomponente aus einem offenporigen Elastomer und ist recht weich, so dass sie sich auch der Form des Weichgewebes im Mund des Patienten anpasst. Die harte Hohlkammer ermöglicht hingegen einen sicheren Anschluss an den Saugschlauch und verhindert, dass der Saugbereich kollabiert.

Die Hohlkammer hat bevorzugt eine in ihrer Längsrichtung leicht gebogene Ausgestaltung. Die Weichkomponente erstreckt sich an der konvexen Seite der

Hohlkammer, so dass diese dort eine ballige Form hat. Diese Lösung ermöglicht eine gute Absenkung an die tiefste Stelle im Oralbereich des Patienten.

Im Querschnitt hat die Hohlkammer eine elliptische oder ovale Form. Dadurch wird sie als schlank und für die Zunge weniger als Fremdkörper empfunden.

Der Querschnitt der Hohlkammer ist wesentlich größer als der Querschnitt des Saugschlauch, beispielsweise etwa fünf bis zehnmal so groß. Er ist bevorzugt über seinen Verlauf konstant.

In vorteilhafter Ausgestaltung der Erfindung weist der konvexe Saugbereich eine Profilstruktur mit Rippen oder Vorsprüngen auf. Durch die Rippen oder Vorsprünge wird die Saugleistung des erfindungsgemäßen Speichelsaugers überraschend vergrößert. Die Saugporen werden nicht durch Anlage an dem Weichgewebe verschlossen.

In weiterer vorteilhafter Ausgestaltung ist ein Zungenhalter vorgesehen, der einstückig mit der Hartkomponente sich nach oben erstreckt, an der der Weichkomponente gegenüberliegenden Seite dieser.

In weiterer bevorzugter Ausgestaltung ist es vorgesehen, dass der Saugschlauch eine integrierte Drahtaufnahme aufweist. Diese bildet einen dünnen Kanal innerhalb des Saugschlauch. Dort ist ein Draht vorgesehen, der bevorzugt mit dem Adapter in Eingriff ist, wenn das Saugelement, also das Kunststoff-Formteil, mit dem Saugschlauch verbunden ist.

Weitere vorteilhafte Merkmale und Einzelheiten ergeben sich aus der nachfolgenden Beschreibung eines Ausführungsbeispiels der Erfindung anhand der Zeichnung.

Es zeigen:
- Fig. 1: eine schematische Ansicht eines erfindungsgemäßen Speichelsaugers ist in fertig montierter Form;
- Fig. 2: die Ausführungsformen gemäß Fig. 1 , jedoch in getrennter Form;
- Fig. 3: einen Adapter für einen erfindungsgemäßen Speichelsauger;
- Fig. 4: eine schematische perspektivische Ansicht eines erfindungsgemäßen Kunststoff-Formteil es für einen erfindungsgemäßen Speichelsauger;
- Fig. 5: eine Schnittansicht durch das Kunststoff-Formteile gemäss Fig. 4;
- Fig. 6: eine perspektivische Ansicht des erfindungsgemäßen Speichelsauger ist unter Darstellung des Endes des Saugschlauch; und
- Fig. 7: eine vergrößerte Darstellung des Endes des Saugschlauchs.

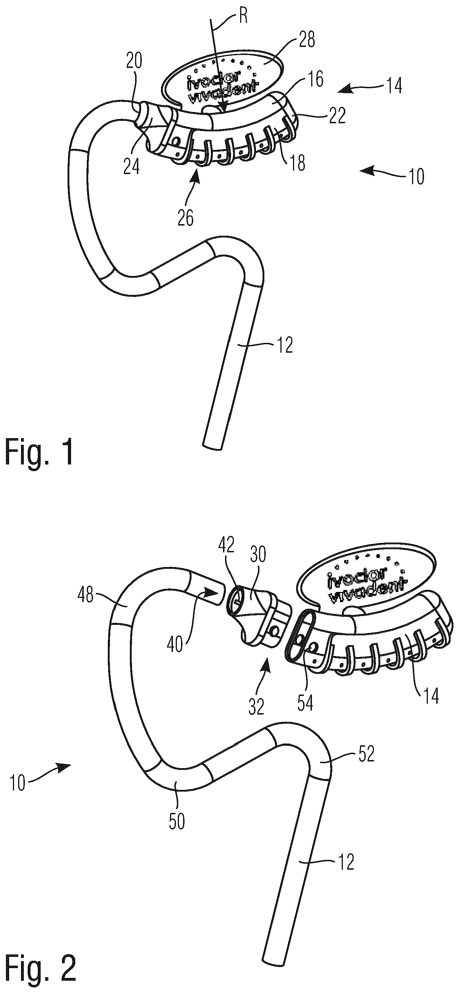

In Fig. 1 ist ein Speichelsauger 10 schematisch dargestellt. Der Speichelsauger 10 weist einen Saugschlauch 12 und ein Kunststoff-Formteil 14 auf, das aus Spritzguss besteht, also per Spritzgießen hergestellt ist.

Das Formteil 14 ist als Mehrkomponenten-Spritzgussteil ausgebildet. Es besteht aus einer harten Kunststoff-Komponente 16 und einer weicheren Kunststoffkomponente 18. Beide sind in einem Arbeitsgang hergestellt und einstückig miteinander verbunden. Die härtere Kunststoff-Komponente 16 kann ein beliebiger geeignete biokompatibler Kunststoff sein, der für den vorgesehenen Anwendungszweck ausreichende Eigenschaften aufweist. Die weichere Kunststoff-Komponente ist ein Kunststoff, der Poren oder kleine Öffnungen aufweist, die für den Durchlass von Flüssigkeiten geeignet sind.

Der Formteil 14 wird durch den Saugschlauch 12 unter Unterdruck gesetzt. Dies führt dazu, dass die Poren oder Öffnungen in der weicheren Kunststoff-Komponente eine Saugfunktion haben.

Beispielsweise kann die weichere Kunststoff-Komponente aus einem offenporigen Elastomer bestehen. Es ist auch möglich, eine weichere Kunststoff-Komponente zu verwenden, die Mikro-Röhren aufweist, die sich in Richtung Inneres des Formteils 14 erstrecken. Die weichere Komponente kann auch in Form eines Schwammes ausgebildet sein. Ferner kann sie auch eine Filterfunktion aufweisen. Wie aus Fig. 1 ersichtlich ist, ist das Formteil 14 leicht gebogen, und hat einen Krümmungsradius, der sich quer zu seiner Längsrichtung erstreckt. Das Formteil 14 ist als Hohlkammer ausgebildet. Es erstreckt sich in Längsrichtung nach der Art eines Zylinders, der allerdings gekrümmt ist. Das Verhältnis zwischen dem Durchmesser dieses gekrümmten Zylinders und der Länge beträgt zwischen 1 zu 2 und 1 zu 10, im dargestellten Ausführungsbeispiel etwa 1 zu 4.

Die härtere Kunststoff-Komponente 16 nimmt den Großteil der Außenwand der Hohlkammer 14 ein. Sie erstreckt sich auch über ein Ende des Formteils 14, das eine Auslassöffnung 20 bildet, und ein gegenüberliegendes Ende 22, das geschlossen ist. An der Auslassöffnung 20 ist eine Kupplung 24 ausgebildet, die dem Anschluss an den Saugschlauch 12 dient.

Zwischen diesen beiden Enden, jedoch nur auf einer Seite des gekrümmten Zylinders des Formteils oder der Hohlkammer 14 ist die weichere Kunststoff-Komponente 18 ausgebildet. Sie stellt zugleich einen Saugbereich 26 bereit. Dieser weist die bereits erwähnten Saugröhren auf und dient dazu, Speichel aufzusaugen. Im Inneren der Hohlkammer 14 besteht ein Unterdruck, der durch den Saugschlauch 12 erzeugt wird.

Die gekrümmt zylindrische Hohlkammer 14 weist einen konvexen Bereich auf, an dem die weichere Komponente angeordnet ist, und einen konkaven Bereich, an dem die härtere Kunststoff Komponente 16 angeordnet ist. Der Querschnitt der Hohlkammer 14 ist im Wesentlichen elliptisch. In den Mund des Patienten eingebracht erstreckt sich die weichere Kunststoff-Komponente 18 nach unten und die härtere Kunststoff-Komponente nach oben. Insofern kann der Querschnitt des Formteils 14 auch als hochelliptisch oder hochoval bezeichnet werden.

An der dem weicheren Kunststoff-Formteil 18 entgegengesetzten Seite ist ein Zungenhalter 28 angeordnet. Dieser ist ebenfalls einstückig mit dem Formteil 14 verbunden und erstreckt sich nach oben, wenn der Speichelsauger 10 in den Mund des Patienten eingebracht ist. Er dient dazu, die Zunge von den Zähnen wegzuhalten.

Durch die weiche Abstützung des Formteils 14 über die Weichkomponente 18 an dem oralen Weichgewebe wird der Zungenschutz nach oben in aufrechter Stellung gehalten, ohne dass dies für den Patienten Schmerzen oder Irritationen verursacht und schützt die zu behandelnden Zähne vor den Aktivitäten der Zunge.

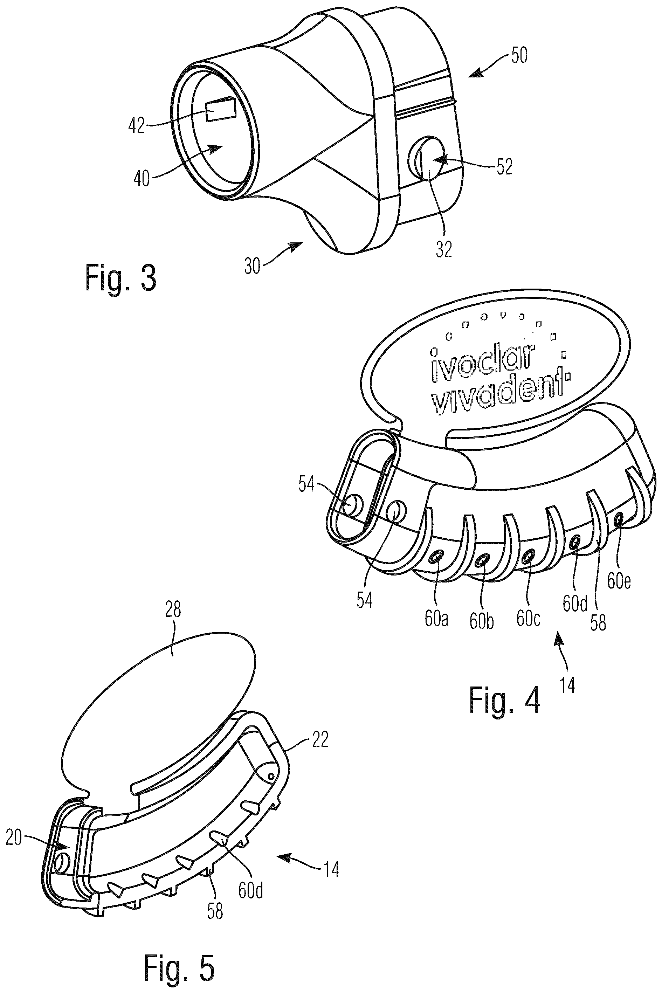

Der Saugschlauch 12 ist lösbar in die Kupplung 24 eingesteckt. Die Kupplung 24 weist wie aus Fig. 3 ersichtlich besondere Maßnahmen auf, die ein unabsichtliches Lösen des Saugschlauchs verhindern.

Aus Fig. 2 sind die Teile des erfindungsgemäßen Speichelsaugers im einzelnen ersichtlich.

Es ist ersichtlich, dass die Kupplung 24 an einem separaten Formteil 30 ausgebildet ist, das zugleich einen Adapter darstellt. Der Adapter 30 ist ebenfalls aus Hartkunststoff ausgebildet, entsprechend der härteren Kunststoff-Komponente 16, und ist über Rastelemente 32 mit dem Formteil 14 verbunden.

Der Adapter 30 weist ferner an seiner Aufnahme-Öffnung 40, die für die Aufnahme des Saugschlauch 12 bestimmt ist, einwärts gewandte Sperrzungen 42 auf. Diese dienen dem formschlüssigen Eingriff in die Außenseite des vergleichsweise weichen Saugschlauch 12. Mit dieser speziell ausgestalteten Aufnahmeöffnung 40 ist der Adapter 30 gleichsam unverlierbar mit dem Saugschlauch 12 verbunden.

Wenn das Formteil 14 ausgetauscht werden soll, wird es bevorzugt über das Lösen der Rastvorrichtung 32 von dem Adapter 30 entkoppelt. Der Adapter 30 verbleibt also an dem Saugschlauch 12, und das gegebenenfalls zu desinfizierende oder zu auszutauschende Formteil 14 kann entsprechend dem gewünschten Verwendungszweck behandelt werden.

Anschließend hieran kann das gleiche oder ein neues Formteil 14 zum Einsatz gelangen.

Wie aus Fig. 1 und 2 ersichtlich ist, ist der Saugschlauch 12 mehrfach gekrümmt. Er weist einen Zahnbogen 48 auf. An dieser Stelle übertritt der Saugschlauch 12 die Zahnreihe des Patienten, oder gegebenenfalls einen Bereich distal des hintersten Molaren des Patienten.

Von dem Zahnbogen 48 ausgehend überwindet der Saugschlauch 12 - etwas verkürzt dargestellt - die Unterlippe des Patienten und verläuft anschließend hieran nach unten. An einem Kinnbogen 50 ist er nach distal abgebogen, also zum Hals des Patienten hin, und dann erneut in Gegenrichtung an einem Halsbogen 52.

Mit dieser Ausgestaltung ist es sichergestellt, dass das Formteil 14 im Mund des Patienten nach unten gedrückt wird, also entweder vestibulär nach unten oder im oralen Bereich nach unten. Das Aufbringen des Drucks erfolgt entweder über den insofern vorgeformten Saugschlauch 12 selbst, oder aber durch einen Draht, der vorgeformt ist und elastisch ist und anhand der Fig.en 6 und 7 näher beschrieben wird.

Aus Fig. 3 ist der Adapter 30 im einzelnen ersichtlich. Gleiche Bezugszeichen weisen hier wie auch in den weiteren Fig.en auf gleiche oder entsprechende Teile hin. Aus Fig. 3 ist die hochovale Ausgestaltung des formteilseitigen Endes 50 des Adapters 30 ersichtlich. Diese entspricht der hochovalen Ausgestaltung des Formteils 14. Die Aufnahmeöffnung 40 weist seitlich die bereits angesprochenen Sperrnasen auf, von dem die Sperrnase 42 aus Fig. 3 ersichtlich ist.

Die Rasteinrichtung 32 zum Formteil 14 hin besteht aus einem Rastvorsprung 52 und einer hierfür geeignet ausgestalteten Öffnung 54 in dem Formteil 14. Der Rastvorsprung 52 tritt in die Rastausnehmung 54 ein und durchtritt diese. Durch Druck auf den Rastvorsprung 52 lässt sich der Adapter 30 von dem Formteil 14 entkoppeln.

Die Aufnahmeöffnung 40 ist so bemessen, dass sich der Saugschlauch 12 in Formschluss in sie einführen lässt. Bevorzugt wird der Saugschlauch 12 an den hierfür geeigneten Stellen kurz eingekerbt, um die Sperrzungen 40 formschlüssig aufnehmen zu können. Dann ist die Verbindung so fest, dass sie sich auch bei einem üblichen Zug nicht löst.

Aus Fig. 4 ist ein Formteil 14 in vergrößerten Darstellung perspektivisch ersichtlich. Im Bereich der Weichkomponente 18 weist das Formteil 14 nach unten vorspringende Rippen 58 auf. Zwischen diesen erstrecken sich zahlreiche Saugporen, von denen Saugporen 60a bis 60e aus Fig. 4 ersichtlich sind. Die Saugporen 60a bis 60e springen gegenüber den Rippen 58 nach oben zurück. Hierdurch wird verhindert, dass das dort anliegende Weichgewebe die Poren Öffnungen abdeckt.

Aus Fig. 5 ist das Formteil 14 im Schnitt ersichtlich. Der Schnitt ist auch durch den Zungenhalter 28 vorgesehen, der dementsprechend keine Beschriftung zeigt. Es ist ersichtlich, dass das Formteil 14 als Hohlkammer 14 ausgebildet ist.

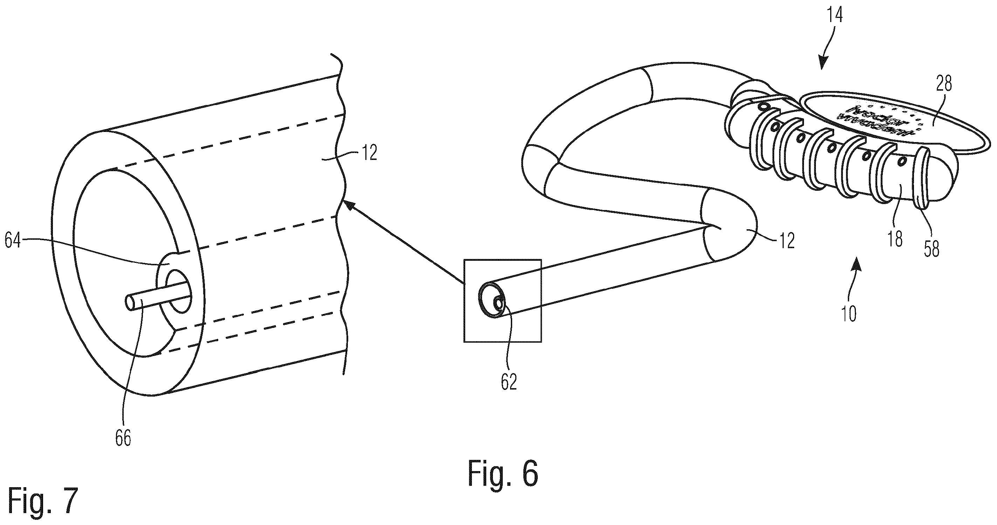

Aus Fig. 6 ist eine demgegenüber etwas modifizierte Ausgestaltung des erfindungsgemäßen Speichelsaugers 10 ersichtlich. Der Unterschied betrifft die Ausgestaltung einer integrierten Drahtaufnahme 62. Diese besteht aus einem aus Fig. 7 besser ersichtlichen Kanal 64. Der Kanal 64 ist einstückig mit dem Saugschlauch 12 ausgebildet und erstreckt sich an dessen Außenrand, mit einem gegenüber dem Durchmesser des Saugschlauchs 12 deutlich geringeren Durchmesser von beispielsweise einem Fünftel bis 1/ 20 des Durchmessers des Saugschlauchs 12.

In den Kanal 64 lässt sich ein Draht 66 einbringen. Der Draht 66 ist dem Grunde nach biegsam, weist jedoch eine gewisse Elastizität auf. Dies führt dazu, dass er den Saugschlauch am Kinn des Patienten andrücken kann und hierdurch das Formteil 14 nach unten drückt.

Der Draht 64 kann auch ausgenutzt werden, um den Adapter 30 besser mit dem Saugschlauch 12 zu verankern. Hierzu ist er in den Adapter 30 in eine entsprechende Öffnung eingeführt und anschließend hieran abgebogen. Hierdurch entsteht ein Formschluss zwischen dem Draht 64 und damit dem Saugschlauch 12 und den Adapter 30.

Insofern kann der Draht 64 eine Doppelfunktion erfüllen.

## Patentansprüche

1. Speichelsauger, mit einem Kunststoff-Formteil (14) aus Spritzguss, das eine Kupplung (24) für den Anschluss an einen Saugschlauch (12) ausgebildet, wobei das Kunststoff-Formteil (14) als Mehrkomponenten-Spritzgussteil hergestellt ist und eine gegenüber einer anderen härtere Kunststoffkomponente, die Hartkomponente (16), die Kupplung (24) ausbildet und die andere Kunststoffkomponente, die Weichkomponente (18), einen Saugbereich mit mindestens einer Saugöffnung - oder Saugpore - (60a - 60e) für Speichel und eine Auslassöffnung (20) zum Saugschlauch (12) hin ausbildet und insbesondere aus einem offenporigen, bevorzugt thermoplastischen, Elastomer besteht, und das Formteil (14) gebogen ist, einen Krümmungsradius aufweist, der sich quer zu seiner Längsrichtung erstreckt, als Hohlkammer ausgebildet ist und sich in Längsrichtung nach der Art eines gekrümmten Zylinders erstreckt, wobei sich die Hartkomponente (16) über ein Ende des Formteils (14), das die Auslassöffnung (20) bildet, und ein gegenüberliegendes Ende (22) erstreckt, das geschlossen ist.

2. Speichelsauger nach Anspruch 1, **dadurch gekennzeichnet, dass** das Kunststoff-Formteil (14) auch einen Zungenhalter (28) ausbildet, der sich insbesondere an der dem Saugbereich (26) gegenüberliegenden Seite des Formteils (14) wegerstreckt.

3. Speichelsauger nach einem der vorhergehenden Ansprüche, **dadurch gekennzeichnet, dass** die Weichkomponente (18) gegenüber dem Saug-Unterdruck des Speichelsaugers (10), insbesondere in Höhe von zwischen 80 mbar und 250 mbar, formstabil ist.

4. Speichelsauger nach einem der vorhergehenden Ansprüche, **dadurch gekennzeichnet, dass** die Hartkomponente (16) sich mindestens teilweise, insbesondere vollständig, um die Auslassöffnung (20) herum erstreckt und diese aufgespannt hält.

5. Speichelsauger nach einem der vorhergehenden Ansprüche, **dadurch gekennzeichnet, dass** der Saugbereich (26) durch eine allseits - abgesehen von Saugporen und/oder Saugöffnungen (60a - 60e) und der Auslassöffnung (20) - geschlossene Hohlkammer ausgebildet ist, die im Betrieb des Speichelsaugers (10) unter Unterdruck steht.

6. Speichelsauger nach einem der vorhergehenden Ansprüche, **dadurch gekennzeichnet, dass** der Saugbereich (26), insbesondere an seiner dem Zungenhalter (28) gegenüberliegenden Seite, vollständig von der Weichkomponente (18) gebildet ist.

7. Speichelsauger nach einem der vorhergehenden Ansprüche, **dadurch gekennzeichnet, dass** die Hartkomponente (16) und die Weichkomponente (18) per Verbundspritzguss miteinander fest verbunden sind.

8. Speichelsauger nach einem der vorhergehenden Ansprüche, **dadurch gekennzeichnet, dass** ein ein Saugschlauch (12) vorgesehen ist und die Auslassöffnung (20) eine gegenüber dem Saugschlauch (12) größeren Querschnitt aufweist und insbesondere einen Querschnitt, der gegenüber einem Kreisquerschnitt in Richtung des Zungenhalters (28) vergrößert ist.

9. Speichelsauger nach einem der vorhergehenden Ansprüche, **dadurch gekennzeichnet, dass** ein Zungenhalter (28) sich in Verlängerung eines hochovalen oder elliptischen Querschnitts des Saugbereichs (26) erstreckt.

10. Speichelsauger nach einem der vorhergehenden Ansprüche, **dadurch gekennzeichnet, dass** ein Saugschlauch (12) vorgesehen ist und der Saugbereich (26) einen über seine Längserstreckung bis zur Auslassöffnung (20) im wesentlichen konstanten Querschnitt aufweist, welcher Querschnitt insbesondere größer als der freie Strömungsquerschnitt des Saugschlauchs (12) ist.

11. Speichelsauger nach einem der vorhergehenden Ansprüche, **dadurch gekennzeichnet, dass** der Saugbereich (26) an seiner dem Zungenhalter (28) oder der Hartkomponente (16) gegenüberliegenden Seite eine Profilstruktur, insbesondere mit Rippen (58) oder Vorsprüngen aufweist, mit einer über den Verlauf der Seite im wesentlichen gleichen Profilhöhe.

12. Speichelsauger nach Anspruch 11, **dadurch gekennzeichnet, dass** die Profilstruktur eine Höhe von mehr als 0,2 mm, insbesondere etwa 0,5mm, aufweist und die Saugporen (60a - 60e) des Saugbereichs (26) am Grund der Profilstruktur enden, so dass insbesondere auch bei Anlage der Profilstruktur an Gewebe die Profilstruktur eine Blockade der Saugpore (60a - 60e) durch das Gewebe verhindert.

13. Speichelsauger nach einem der vorhergehenden Ansprüche, **dadurch gekennzeichnet, dass** ein Adapter (30) an die Kupplung (24) des Formteils (14) lösbar ankuppelbar ist und insbesondere eine Querschnittsanpassung zwischen dem Saugschlauch (12) und der Auslassöffnung (20) herstellt und aus der Hartkomponente (16) hergestellt ist, insbesondere als gegenüber dem Formteil (14) separates Teil.

14. Speichelsauger nach Anspruch 13, **dadurch gekennzeichnet, dass** der Adapter (30) über die Kupplung (24) formschüssig mit dem Kunststoff-Formteil (14) und/oder formschlüssig mit dem Saugschlauch (12) verbindbar ist oder verbunden ist.

15. Speichelsauger nach einem der vorhergehenden Ansprüche, **dadurch gekennzeichnet, dass** ein Saugschlauch (12) vorgesehen ist, der eine integrierte Drahtaufnahme (62) für die Aufnahme eines plastisch verformbaren Drahtes (66) aufweist, die an dem Adapter (30) und/oder an dem Kunststoff-Formteil (14) für die Verbindung des Drahtes (66) mit diesem endet.

16. Speichelsauger nach Anspruch 15, **dadurch gekennzeichnet, dass** der Draht (66) sich in den Adapter (30) hinein erstreckt und in diesem unverlierbar verankert ist, so dass insbesondere der Saugschlauch (12) nicht zerstörungsfrei von dem Adapter (30) trennbar ist.

## Claims

1. A saliva aspirator, having a plastic moulded part (14) made of injection moulding, which forms a coupling (24) for connection to a suction hose (12), wherein the plastic moulded part (14) is produced as a multi-component injection-moulded part and forms the coupling (24) with respect to a harder plastic component, the hard component (16), and the other plastic component, the soft component (18), forms a suction region with at least one suction opening - or suction pore - (60a - 60e) for saliva and an outlet opening (20) towards the suction hose (12) and consists in particular of an open-pored, preferably thermoplastic, elastomer, and wherein the moulded part (14) is bent, has a radius of curvature extending transversely to its longitudinal direction, is configured as a hollow chamber and extends in the longitudinal direction according to the type of a curved cylinder, wherein the hard component (16) extends across an end of the moulded part (14) which forms the outlet opening (20), and an opposite end (22) which is closed.

2. The saliva aspirator according to claim 1, **characterised in that** the plastic moulded part (14) also forms a tongue holder (28) which extends away in particular on the side of the moulded part (14) opposite the suction region (26).

3. The saliva aspirator according to one of the preceding claims, **characterized in that** the soft component (18) is dimensionally stable with respect to the suction vacuum of the saliva aspirator (10), in particular at a level of between 80 mbar and 250 mbar.

4. The saliva aspirator according to one of the preceding claims, **characterized in that** the hard component (16) extends at least partially, in particular completely, around the outlet opening (20) and keeps it stretched open.

5. The saliva aspirator according to one of the preceding claims, **characterised in that** the suction region (26) is formed by a hollow chamber which is closed on all sides - apart from suction pores and/or suction openings (60a - 60e) and the outlet opening (20) - and which is under negative pressure during operation of the saliva aspirator (10).

6. The saliva aspirator according to one of the preceding claims, **characterised in that** the suction region (26), in particular on its side opposite the tongue holder (28), is formed entirely by the soft component (18).

7. The saliva aspirator according to one of the preceding claims, **characterised in that** the hard component (16) and the soft component (18) are firmly connected to each other by bonded injection moulding.

8. The saliva aspirator according to one of the preceding claims, **characterised in that** a suction hose (12) is provided and that the outlet opening (20) has a cross-section which is larger than that of the suction hose (12) and, in particular, a cross-section which is larger than a circular cross-section in the direction of the tongue holder (28).

9. The saliva aspirator according to one of the preceding claims, **characterised in that** a tongue holder (28) extends in extension of a highly oval or elliptical cross-section of the suction region (26).

10. The saliva aspirator according to one of the preceding claims, **characterised in that** a suction hose (12) is provided and that the suction region (26) has a cross-section which is substantially constant over its longitudinal extension up to the outlet opening (20), which cross-section is in particular larger than the free flow area of the suction hose (12).

11. The saliva aspirator according to one of the preceding claims, **characterised in that** the suction region (26) has, on its side opposite the tongue holder (28) or the hard component (16), a profiled structure, in particular with ribs (58) or projections, with a profile height which is substantially the same over the course of the side.

12. The saliva aspirator according to claim 11, **characterised in that** the profile structure has a height of more than 0.2 mm, in particular about 0.5 mm, and the suction pores (60a - 60e) of the suction region (26) end at the base of the profile structure, so that, in particular also when the profile structure bears against tissue, the profile structure prevents the suction pore (60a - 60e) from being blocked by the tissue.

13. The saliva aspirator according to one of the preceding claims, **characterised in that** an adapter (30) can be releasably coupled to the coupling (24) of the moulded part (14) and, in particular, produces a cross-sectional adaptation between the suction hose (12) and the outlet opening (20) and is produced from the hard component (16), in particular as a part separate from the moulded part (14).

14. The saliva aspirator according to claim 13, **characterised in that** the adapter (30) can be or is positively connected to the plastic moulded part (14) and/or positively connected to the suction hose (12) via the coupling (24).

15. The saliva aspirator according to one of the preceding claims, **characterised in that** a suction hose (12) is provided which has an integrated wire receptacle (62) for receiving a plastically deformable wire (66), which ends at the adapter (30) and/or at the plastic moulded part (14) for connecting the wire (66) thereto.

16. The saliva aspirator according to claim 15, **characterised in that** the wire (66) extends into the adapter (30) and is undetachably anchored therein, so that in particular the suction hose (12) cannot be separated from the adapter (30) without being destroyed.

## Revendications

1. Aspirateur de salive, comprenant une pièce moulée en plastique (14) obtenue par moulage par injection, qui forme un raccord (24) pour le raccordement à un tuyau d'aspiration (12), où la pièce moulée en plastique (14) est fabriquée sous forme de pièce moulée par injection à plusieurs composants et, par rapport à un composant en plastique plus dur, le composant dur (16), forme le raccord (24), et l'autre composant en plastique, le composant souple (18), forme une zone d'aspiration avec au moins une ouverture d'aspiration - ou un pore d'aspiration - (60a - 60e) pour la salive et un orifice de sortie (20) vers le tuyau d'aspiration (12) et est en particulier constitué d'un élastomère à pores ouverts, de préférence thermoplastique, et la pièce moulée (14) est courbée, présente un rayon de courbure qui s'étend transversalement à sa direction longitudinale, est conçu comme une chambre creuse et s'étend dans le sens longitudinale à la manière d'un cylindre courbé, où le composant dur (16) s'étend au-delà d'une extrémité de la pièce moulée (14) qui forme l'ouverture de sortie (20) et une extrémité opposée (22) qui est fermée.

2. Aspirateur de salive selon la revendication 1, **caractérisé en ce que** la pièce moulée en plastique (14) forme également un support de la langue (28) qui s'étend en particulier du côté de la pièce moulée (14) opposé à la zone d'aspiration (26).

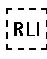 3. Aspirateur de salive selon l'une des revendications précédentes, **caractérisé en ce que** le composant souple (18) est indéformable face à la dépression d'aspiration de l'aspirateur de salive (10), en particulier à une hauteur comprise entre 80 mbar et 250 mbar.

4. Aspirateur de salive selon l'une des revendications précédentes, **caractérisé en ce que** le composant dur (16) s'étend au moins partiellement, en particulier complètement, autour de l'ouverture de sortie (20) et maintient celle-ci ouverte.

5. Aspirateur de salive selon l'une des revendications précédentes, **caractérisé en ce que** la zone d'aspiration (26) est formée par une chambre creuse fermée de tous côtés, à l'exception des pores d'aspiration et/ou des ouvertures d'aspiration (60a à 60e) et l'ouverture de sortie (20), qui est sous vide pendant le fonctionnement de l'aspirateur de salive (10).

6. Aspirateur de salive selon l'une des revendications précédentes, **caractérisé en ce que** la zone d'aspiration (26), en particulier sur son côté opposé au support de la langue (28), est entièrement formée par le composant souple (18).

7. Aspirateur de salive selon l'une des revendications précédentes, **caractérisé en ce que** le composant dur (16) et le composant souple (18) sont solidement reliés entre eux par moulage par injection composite.

8. Aspirateur de salive selon l'une des revendications précédentes, **caractérisé en ce qu'**un tuyau d'aspiration (12) est prévu et que l'ouverture de sortie (20) présente une section transversale plus grande que celle du tuyau d'aspiration (12) et, en particulier, une section transversale qui est agrandie par rapport à une section transversale circulaire en direction du support de langue (28).

9. Aspirateur de salive selon l'une des revendications précédentes, **caractérisé en ce qu'**un support de la langue (28) s'étend dans le prolongement d'une section transversale ovale ou elliptique de la zone d'aspiration (26).

10. Aspirateur de salive selon l'une des revendications précédentes, **caractérisé en ce qu'**un tuyau d'aspiration (12) est prévu et que la zone d'aspiration (26) présente une section transversale sensiblement constante sur toute sa longueur jusqu'à l'ouverture de sortie (20), laquelle section transversale étant en particulier plus grande que la section transversale libre d'écoulement du tuyau d'aspiration (12).

11. Aspirateur de salive selon l'une des revendications précédentes, **caractérisé en ce que** la zone d'aspiration (26) présente, sur son côté opposé au support de la langue (28) ou au composant dur (16), une structure profilée, en particulier avec des nervures (58) ou des saillies, avec une hauteur de profil sensiblement identique sur toute la longueur du côté.

12. Aspirateur de salive selon la revendication 11, **caractérisé en ce que** la structure profilée présente une hauteur supérieure à 0,2 mm, en particulier d'environ 0,5 mm, et que les pores d'aspiration (60a - 60e) de la zone d'aspiration (26) se terminent à la base de la structure profilée, de sorte que, en particulier lorsque la structure profilée est appliquée sur un tissu, la structure profilée empêche le tissu de bloquer les pores d'aspiration (60a - 60e).

13. Aspirateur de salive selon l'une des revendications précédentes, **caractérisé en ce qu'**un adaptateur (30) peut être couplé de manière amovible au raccord (24) de la pièce moulée (14) et réalise en particulier une adaptation de section entre le tuyau d'aspiration (12) et l'ouverture de sortie (20) et est fabriqué à partir du composant dur (16), en particulier en tant que pièce séparée de la pièce moulée (14).

14. Aspirateur de salive selon la revendication 13, **caractérisé en ce que** l'adaptateur (30) peut être relié ou est relié par complémentarité de forme à la pièce moulée en plastique (14) et/ou par complémentarité de forme au tuyau d'aspiration (12) via le raccord (24).

15. Aspirateur de salive selon l'une des revendications précédentes, **caractérisé en ce qu'**un tuyau d'aspiration (12) est prévu qui comporte un logement de fil intégré (62) pour recevoir un fil plastiquement déformable (66), qui se termine sur l'adaptateur (30) et/ou sur la pièce moulée en plastique (14) pour relier le fil métallique (66) à celuici.

16. Aspirateur de salive selon la revendication 15, **caractérisé en ce que** le fil métallique (66) s'étend dans l'adaptateur (30) et y est ancré de manière imperdable, de manière à ce que le tuyau d'aspiration (12) en particulier ne puisse pas être séparé de l'adaptateur (30) sans être endommagé.
